# EUROPEAN PATENT APPLICATION

(11) **EP 3 569 716 A1**
(43) Date of publication of application: **20.11.2019**
(21) Application number: 18382329.3
(22) Date of filing: 14.05.2018
(51) Int. Cl.: C12Q 1/686, G01N 27/00, G01N 21/77, A61B 5/00, G01N 33/487, B01L 3/00, G01N 33/543, G01N 33/558, G01N 21/75, G01N 21/01

(54) **A METHOD FOR CONTROLLING TIMING OF EVENTS IN A MICROFLUIDIC DEVICE AND A TIMER MICROFLUIDIC DEVICE**

(71) Applicant: Consejo Superior De Investigaciones Científicas (CSIC), 28006 Madrid (ES); Institució Catalana De Recerca I Estudis Avançats (ICREA), 08010 Barcelona (ES); Fuelium, SL, 08193 Cerdanyola del Vallès (ES)
(72) Inventor: ESQUIVEL BOJORQUEZ, Juan Pablo, 08006 Barcelona (ES); SABATÉ VIZCARRA, Maria de les Neus, 08014 Barcelona (ES); GASSÓ PONS, Sergi, 17178 Sant Privat d'en Bas (ES)
(74) Representative: Juncosa Miró, Jaime

(57) **Abstract**

The method comprises adding a liquid on a first end of a microfluidic device at a first time t₀, the liquid flowing by capillarity towards a second end; producing, by a battery (12) included in the microfluidic device, energy from a second time tₛₜₐᵣₜ until a third time t_{end} to feed an auxiliary device (16) connected to the battery (12), said tₛₜₐᵣₜ corresponding to the moment when the liquid contacts the battery (12); using a delivery energy time interval tₒₚₑᵣₐₜᵢₒₙ of the battery (12) comprised between a time tₒₙ in which a voltage output of the battery (12) is above a threshold and a time t_{off} in which the voltage output is below the threshold to control the duration of an event including a selective activation and deactivation of said auxiliary device (16), wherein the battery (12) being sized and composed to provide a given amount of energy.

## Description

### Technical Field

The present invention is directed, in general, to the field of microfluidic devices. In particular, the invention relates to a method for controlling timing of events in a microfluidic device and to a timer microfluidic device.

A microfluidic device will be understood here as an integrated system within the micrometer scale that comprises a set of tools or elements to operate or interact with liquid samples (filters, valves, mixers, splitters, gradient generator, sample injection, sample concentration, sample separation, heater, cooler, electrodes...). These systems can be used for chemical synthesis, protein crystallization, chemical/biochemical reactor, sample treatment and sample analysis. When the purpose is to prepare, to pretreat, to process and to analyze a sample, they are called microfluidic analytical devices, and can be used in point-of-care applications such as clinical human diagnostics, veterinary diagnostics, environmental analysis, food quality and safety control, biohazard control, among others. A microfluidic device can be made out of polymer, glass, ceramic, paper, wax, silk chitosan, and other organic compounds.

### Background of the Invention

The most widely used technologies for in vitro diagnostics (IVD) is the lateral flow immunoassay. This is mostly because they have a simple test design, they are compact, results are quick and easy to read, and their manufacturing is easy and inexpensive.

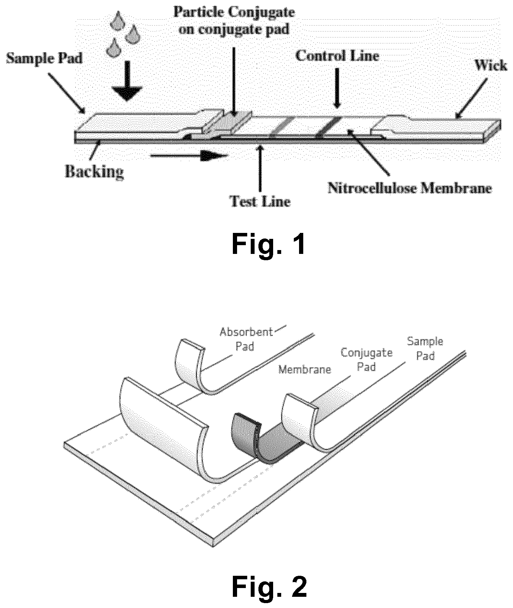

The first tests were made for the detection of human chorionic gonadotropin (hCG), as pregnancy test. Today, there are commercially available tests for monitoring ovulation, detecting infectious disease organisms, analyzing drugs of abuse, and measuring other analytes important to human physiology. Products have also been introduced for veterinary testing, agricultural applications, environmental testing, and product quality evaluation. Fig. 1 shows typical configurations of a lateral flow test.

A lateral flow assay consists of different overlapped porous membranes. The sample is added on the sample pad and flows by capillarity towards the wick or absorbent pad. The conjugate pad contains colored particles conjugated with an antigen or antibody, these particles are re-dissolved with the sample and flow together to the nitrocellulose membrane. The nitrocellulose contains two regions onto which other specific biological components have been immobilized. These are typically proteins, either antibody or antigen, which have been laid down in bands in specific areas of the membrane where they serve to capture the analyte and the conjugate as they flow over the capture lines. Excess reagents move past the capture lines and are entrapped in the wick or absorbent pad. Results are interpreted on the reaction zone in the nitrocellulose membrane as the presence or absence of lines (test line and control line), these can be read either by eye or using a reader.

The different porous membranes comprising the lateral flow are assembled over a backing card with a pressure sensitive adhesive (PSA) (Fig. 2). Then, the whole assembly is cut in individual strips. Sometimes the strips are placed inside a cassette that provides a sample container, a buffer inlet if needed and a window to see the results area on the nitrocellulose strip. The cassette can hold one or multiple test strips inside.

Great efforts are put into development and optimization of diagnostic devices as millions of tests are deployed in the field. A significant part of the tests is faulty due to lack of protocol compliance, such as the use of a timer. It is known that for a typical test procedure for HIV detection in blood samples the manufacturer of the test advices to read the results in just 20 minutes. This seems to be a very easy to follow instruction, however, when these tests are performed in countries or zones with poor resource settings, it is difficult to get a watch to control this time. As a result, many tests yield false negative results, meaning that infected people are diagnosed as healthy individuals just because the test reading was performed before 20 minutes. On the other side, a negative test may turn into a false positive if the reading is taken too late.

US-A1-2016231251 relates to assay test devices such as lateral flow devices or micro fluidic cells on paper, methods and kits for use to monitor, sense, read and display results by using devices with printed electronics, such as batteries, reading devices, and other circuitry and/or using colorimetric means for testing by using a sensitive indicator pH dye, or both.

EP-A1-2932696 discloses an assay apparatus comprising an assay module adapted to perform an assay; and a portable frame adapted to releasably retain the assay module. The assay module comprises a sample receiver and an assay device operatively associated with the sample receiver. In some embodiments, the assay apparatus further comprises at least one functional module releasably retained by the portable frame. The functional module is operatively associated with the assay module when retained by the portable frame.

US-B2-8921118 discloses a paper-based microfluidic system and methods of making the same. In particular, US-B2-8921118 relates to a method of controlling the movement of a fluid sample through the paper-based microfluidic system. The method comprises applying an electric current to the conductive material on the assay device and contacting the main channel region with a fluid sample, wherein applying the electric current to the conductive material prevents the fluidic flow of the sample from the main channel region to the assay region. In some embodiments, applying the electric current evaporates at least a portion of the fluid sample and concentrates an analyte at the boundary of the main channel and the portion of the conductive material disposed across the main channel region.

Apart from that, [1, 2, 3] reviewed different architectures and uses of microfluidic devices. For instance, [4] developed a microfluidic device with parallel microchannels, valves and reaction chambers for protein crystallization. [5] described the use of inertial forces within microfluidic structures for particle focusing, ordering and separation applications. [6] described a biomimetic multilayer microfluidic device that reproduces complex organ-level lung functions responses, for clinical studies, drug screening and toxicology applications.

However, none of these prior art documents discloses a method for controlling timing of events in a microfluidic device (e.g. a lateral flow assay device, a point-of-care microfluidics, etc.) to perform a selective activation and deactivation of an auxiliary device connected to a battery included in the microfluidic device, for example, acting as a visual and/or audible indicator or to generate heat that can assist in some of the test evaluation.

### References:

[1] Stephen R. Quake et al. "Integrated nanoliter systems", Nature Biotechnology, vol. 21, number 10, October 2003.
[2] George M. Whitesides "The origins and the future of microfluidics", Nature, vol. 442|27, July 2006.
[3] Eric K. Sackmann et al. "The present and future role of microfluidics in biomedical research", Nature, vol. 507, March 2014.
[4] Carl L. Hansen et al. "A robust and scalable microfluidic metering method that allows protein crystal growth by free interface diffusion", PNAS, vol. 99, no. 26, December 2002.
[5] Dino Di Carlo et al. "Continuous inertial focusing, ordering, and separation of particles in microchannels", PNAS, vol. 104, no. 48, November 2007.
[6] Donald E. Ingber et al. "Reconstituting Organ-Level Lung Functions on a Chip", Science, vol. 328, June 2010.

### Description of the Invention

In accordance with the present disclosure, provided is, according to a first aspect, a method for controlling timing of events in a microfluidic device comprising adding an amount of liquid on a first end of a microfluidic device at a first time t₀, the liquid flowing by capillarity towards a second end of the microfluidic device; producing, by a battery included in the microfluidic device, energy from a second time tₛₜₐᵣₜ until a third time t_{end} to feed an auxiliary device connected to the battery, said second time tₛₜₐᵣₜ corresponding to the moment when the battery becomes in contact with the liquid; and using a delivery energy time interval tₒₚₑᵣₐₜᵢₒₙ of the battery comprised between a time tₒₙ in which a voltage output of the battery is above a threshold voltage and a time t_{off} in which the voltage output is below the threshold voltage to control the duration of an event including a selective activation and deactivation of said auxiliary device connected to the battery.

According to the proposed method, the battery, which can be positioned/mounted at different regions within the microfluidic device such as in a middle region thereof, in parallel, on a sample pad, etc. is sized and composed to provide a given amount of energy providing the cited delivery time interval tₒₚₑᵣₐₜᵢₒₙ.

In a preferred embodiment, the battery comprises a paper-based battery that is composed of a paper in contact with at least two electroactive electrodes, at least one of them oxidizing (anode) and at least one of them reducing (cathode). The anode electrode can be composed of any redox species, metal, alloy or polymer oxidizing material, for example of anthraquinone, viologen, TEMPO, Calcium, Iron, Sodium, Potassium, Magnesium, Zinc, Aluminum, among others. The cathode electrode can be composed of any redox species, metal, alloy or polymer reducing material, for example of an air-breathing cathode, Manganese, Iron, Cobalt, Nickel, benzoquinone, TEMPO, among others. That is, in this case the battery generates energy from the oxidation of the anode and a reduction reaction at the cathode. The battery decreases its performance as the electrodes are consumed and its reaction stops when at least one of the electrodes is completely consumed.

Moreover, the microfluidic device may comprise a set of tools or elements to operate or interact with liquid samples, that can include a network of channels and chambers, valves or pumps to control and manipulate fluids to perform different operations such as detection or sample preparation. The microfluidic device can sometimes require an external power source to perform its functions. In some cases, the microfluidic device can include a blister with liquid buffers or other substances required for the device operation. The microfluidic device can be made out of polymer, glass, ceramic, paper, wax, silk chitosan, and other organic compounds. These systems can be used for chemical synthesis, protein crystallization, chemical/biochemical reactor, sample treatment and sample analysis. As previously indicated when the purpose is to prepare, to pretreat, to process and to analyze a sample, the microfluidic devices are called microfluidic analytical devices and can be used in point-of-care applications such as clinical human diagnostics, veterinary diagnostics, environmental analysis, food quality and safety control, biohazard control, among others.

In a preferred embodiment, the microfluidic device comprises a microfluidic analytical device including a lateral flow assay device. In this particular case, the liquid comprises a liquid sample and the device further includes a sample pad located at the first end and a lateral flow test strip through which the liquid flows by capillarity.

It might be an adjustable time interval t_{delay} between said first time t₀ and the delivery energy time interval tₒₚₑᵣₐₜᵢₒₙ. In the particular case of the microfluidic device being a lateral flow assay, the time interval t_{delay} can be adjusted, for example, by modifying the length of the paper strip that transports the liquid sample from the first end to the battery. The longer the strip, the longer the time interval t_{delay}.

According to this invention the auxiliary device when activated during the delivery energy time interval tₒₚₑᵣₐₜᵢₒₙ indicates an enabling time in which a result for example of an assay has to be taken.

The delivery energy interval tₒₚₑᵣₐₜᵢₒₙ can be modified/adjusted. In a first embodiment, this is done by connecting an electric discharge load either passive (like a resistor) or active (like a circuit) to the battery. In a second embodiment, this is done by modifying the active area (i.e. anode and cathode area) of the battery. In a third embodiment, this is done by modifying a thickness of the anode of the battery.

Moreover, in an embodiment, an electrical circuit such as a transistor or an operational amplifier can be used to switch on/off the delivering of power of the battery when a given voltage level (or current level) is reached.

In accordance with the present disclosure, provided also is, according to a second aspect, a timer microfluidic device, comprising a first end adapted to receive an amount of liquid at a first time t₀, the microfluidic device having a second end towards the liquid (1) flows by capillarity, and a battery configured to produce energy when in contact with the liquid, from a second time tₛₜₐᵣₜ until a third time t_{end} to feed an auxiliary device connected to the battery.

The battery is sized and composed to provide a given amount of energy to control the duration of an event including a selective activation and deactivation of said auxiliary device connected to the battery, during a delivery energy time interval tₒₚₑᵣₐₜᵢₒₙ of the battery comprised between a time tₒₙ in which a voltage output of the battery is above a threshold voltage and a time t_{off} in which the voltage output is below the threshold voltage.

The auxiliary device may comprise a lighting system including a Light Emitting Diode (LED), an audible system such as a loudspeaker, a buzzer or an alarm, among others, and/or a device transmitting a radiofrequency signal.

Alternatively, the auxiliary device can comprise a window that is enabled for a vision therethrough during the delivery energy time interval tₒₚₑᵣₐₜᵢₒₙ and that is disabled thereafter. For example, the window can be opened to indicate that the result of a test is valid. The window can include a mechanical window, a liquid crystal dispersion film or an electrochromic film, among others.

Even, the auxiliary device can comprise a heater that is heated up until a given temperature during the delivery energy time interval tₒₚₑᵣₐₜᵢₒₙ. The heater can be also used to perform other functions such as cellular lysis or nucleic acid amplification.

In an embodiment, the microfluidic device is placed inside a container, or cassette, made of plastic, a polymeric material, a wax, among others. The container may further include several additional devices to cooperate with the microfluidic device functions including an electrical discharge load including a resistor or a digital or analog circuit, as well as switches.

### Brief Description of the Drawings

The previous and other advantages and features will be more fully understood from the following detailed description of embodiments, with reference to the attached figures, which must be considered in an illustrative and non-limiting manner, in which:
Fig. 1 is a schematic view of a lateral flow test strip according to the state of the art.
Fig. 2 is a schematic illustration of the lamination of materials for lateral flow fabrication as per the state of the art.
Fig. 3 is a flow chart illustrating a method for controlling timing of events in a microfluidic device, according to an embodiment of the invention.
Fig. 4 graphically illustrates the timeline operation of the battery included in the microfluidic device.
Fig. 5 illustrates an example of a LED powered by the battery as a visual indicator of valid time to read result of a test/assay.
Fig. 6 illustrates an embodiment of the proposed timer microfluidic device.

### Detailed Description of Preferred Embodiments

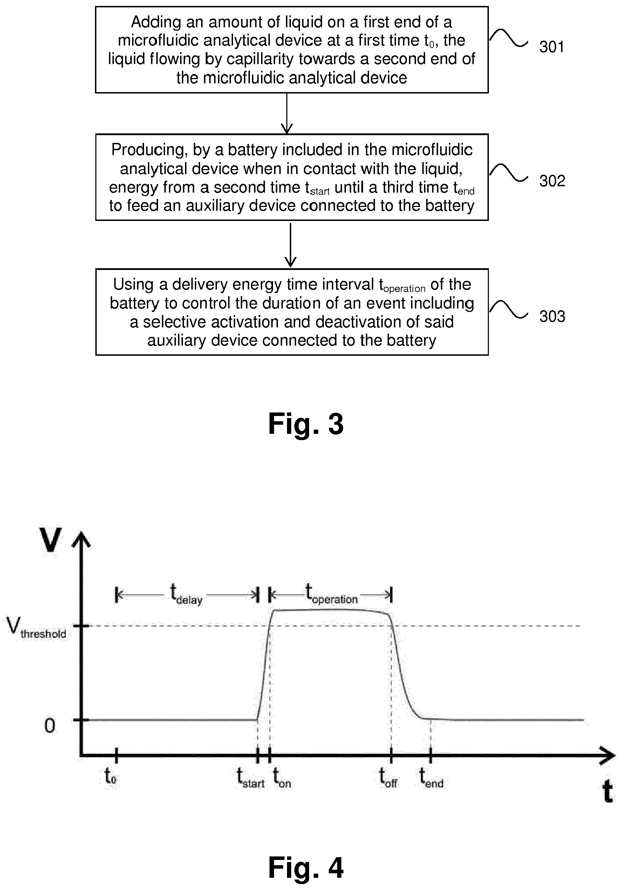

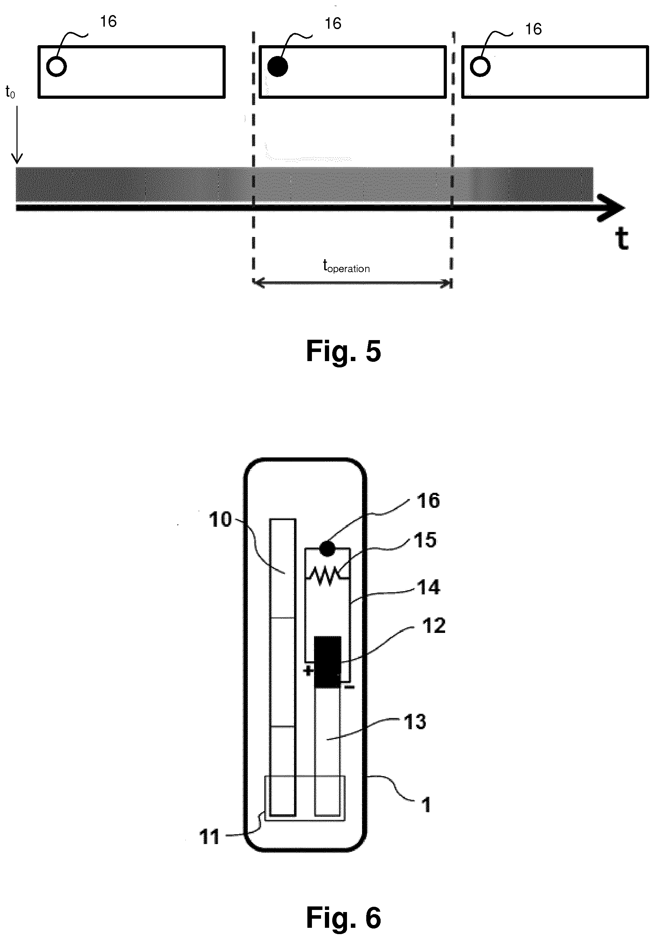

With reference to Fig. 3 therein it is illustrated the basic steps of a method for controlling timing of events in a microfluidic device according to the invention. According to this embodiment, in the method, step 301, a given amount of liquid is added on a first end (or inlet end) of the microfluidic device at a first time t₀, the liquid flowing by capillarity towards a second end, e.g. an outlet end, of the microfluidic device. Then, step 302, when a battery 12 (for example as seen in Fig. 6) included in the microfluidic device becomes in contact with the liquid, the battery starts producing energy from a second time tₛₜₐᵣₜ until a third time t_{end} to feed an auxiliary device 16 connected to the battery 12. At step 303, a delivery energy time interval tₒₚₑᵣₐₜᵢₒₙ of the battery 12 comprised between a time tₒₙ in which a voltage output of the battery 12 is above a threshold voltage and a time t_{off} in which the voltage output is below the threshold voltage is used to control the duration of an event including a selective activation and deactivation of said auxiliary device 16.

Therefore, the battery 12 is a primary battery that is activated upon the addition of a liquid. The performance of the battery 12 in time is illustrated in Fig. 4. As can be seen in the figure, the battery 12 only start producing power after the moment it is wetted (tₛₜₐᵣₜ) and it stops producing power when it is discharged (tend). The energy/power produced by the battery 12 can be used by one or more auxiliary devices 16 (see Fig. 5), which would turn on, for example, when the voltage output of the battery 12 is above the given threshold voltage (V_{threshold}). The period of time when the battery 12 is producing a voltage above the threshold defines the operation time (tₒₚₑᵣₐₜᵢₒₙ), which goes from tₒₙ to t_{off}.

Preferably, the battery 12 comprises a paper-based battery with a metal-based anode (e.g. of Magnesium, Zinc, Aluminum, Lithium, stainless steel, composites, etc.) and an air-breathing cathode. The battery 12 is sized and composed to provide a given amount of energy (related to the duration of the time event to control) and can be fabricated following the same strategies and processes of a lateral flow assay: assembling different layers on a substrate and then cutting them transversally to generate multiple batteries. With this strategy, the battery 12 could be mounted on top of a lateral flow assay in a very simple and cheap way.

When the battery 12 is integrated in an assay, i.e. the microfluidic analytical device comprises a lateral flow assay device, the liquid which comprises a liquid sample is added at time t₀, and there might be a time interval, adjustable, before the liquid reaches the battery (t_{delay}). The delay time can be adjusted, for example, by modifying the length of the paper strip 13 that transports the liquid sample from the sample pad 11, located at the first end, see Fig. 5, to the battery 12.

Several configurations are possible to mount the battery 12 with respect to the microfluidic device. For example, the battery can be positioned on a sample pad 11, on a sink pad, at the backside of the microfluidic device, or in parallel thereof. Following table describes the pros and cons of each configuration.

**Table 1. Examples of battery configurations**

| Position of battery | PROS | CONS |
|---|---|---|
| Sample pad | Energy from the battery is produced from the moment the liquid sample is added. | The by-products of the battery reaction might affect the operation of the assay. |
| Sink pad | By-products of battery reaction do not affect the assay. | The flow rate of sample in the battery and the filling time is limited by the assay membrane materials. |
| | The battery can provide a signal of the moment when the liquid sample has reached the pad. | |
| | Easy to include in the assay. | |
| Backside | Does not interfere with the assay. | It may be more expensive to integrate. |
| | It can be fabricated independently of the assay and combined during final assembly. | |
| | The battery can take advantage of the whole length of the assay. | |
| Parallel | Battery is fabricated completely independent from the assay. | The battery has to be connected to the assay afterwards which may lead to higher production costs. |
| | The battery can be fabricated with less design restrictions. | |

Delivery energy time interval tₒₚₑᵣₐₜᵢₒₙ of the battery 12 can be modified using several strategies, alone or in combination, for example:
▪ By means of an electric discharge load. The value of the electrical passive (like a resistor) or active load (like a circuit or other elements) applied to the battery 12 determines the electric current and, therefore, the rate of discharge of the battery 12. The discharge curve of the battery 12 will be affected by the value of the discharge load, so that the discharge time of the battery 12 is reduced by decreasing the nominal value of the discharge load (or increasing high currents).
▪ Modifying the active area of the battery 12. The amount of electrical current that a battery can produce is proportional to its active area (anode and cathode area). Therefore, increasing the electrode area increases the discharge time of the battery 12 working under the same resistance value.
▪ Modifying the anode thickness of the battery 12. The amount of anode material, which is the material that is consumed during the electrochemical reaction, will determine the operation time of the battery 12. Once the anode is consumed, the battery 12 stops working. The higher the thickness of the anode, the more available material to be consumed and, therefore, the longer discharge times of the batteries.

To control more precisely operation time of the battery 12, an electrical circuit, e.g. electrical switches using transistors or operational amplifiers (not shown), can be used as a switch to start or terminate the delivering of power (electric charge) when the battery 12 reaches a given voltage or current level.

With reference to Fig. 5, therein it is illustrated an embodiment in which the auxiliary device 16 comprises a lighting system such as a LED. The LED can be used to help the user of an assay to know the period when the test is valid to be read. The LED would indicate the user of the test to read the results after the LED has switched off. That is, in this example, the LED would only be ON during the tₒₚₑᵣₐₜᵢₒₙ period of the battery 12. Alternatively, the auxiliary device 16 can comprise an audible system such as a loudspeaker, a buzzer or an alarm, and/or a device transmitting a radiofrequency signal.

In another embodiment, the electrical energy provided by the battery 12 can be used to power a window as auxiliary device 16. The window can be maintained closed and only be opened when the result of the test is valid (adjusting tₒₚₑᵣₐₜᵢₒₙ to this valid time range). The window can be a mechanical window, a liquid crystal dispersion film, electrochromic film or any other.

In yet another embodiment, the electrical energy provided by the battery 12 can be used to generate heat by means of a heater as auxiliary device 16. The heater would behave as a resistive load connected to the battery 12, which contributes to the battery discharging. Therefore, the battery operation time and heater temperature would need to be properly adjusted. The heater temperature could be predefined during device design and fabrication using technologies such as positive temperature coefficient (PTC) heaters. Another way to control the temperature is combining the heater with a phase change material, which is capable of storing a large amount of thermal energy, sustaining a predefined temperature before melting. This particular embodiment can be of great importance in the lateral flow industry as in this industry there is a need to heat up the test to 37 °C in order to improve test reproducibility and to enhance its sensitivity. The heater could also be used to perform other functions in the test, such as cellular lysis or nucleic acid amplification.

With reference now to Fig. 6, the microfluidic device is arranged inside a casing 1, or cassette, to provide robustness and facilitate addition of the liquid sample and reading of the result. The casing 1 can be made of plastic or other materials such as a polymeric material or a wax.

The casing 1 can incorporate some or all of the components involved in the present invention, such as the battery 12, auxiliary device 16, conducting tracks 14, an electrical discharge load 15. Some of these components could be fabricated using manufacturing technologies such as 3D electronics, printed thermoformed electronics, among others.

It should be apparent to those skilled in the art that the description and figures are merely illustrative and not limiting. They are presented by way of example only.

The scope of the present invention is defined in the following set of claims.

## Claims

1. A method for controlling timing of events in a microfluidic device, the method comprising:
adding an amount of liquid on a first end of a microfluidic device at a first time t₀, the liquid flowing by capillarity towards a second end of the microfluidic device;
producing, by a battery (12) included in the microfluidic device, energy from a second time tₛₜₐᵣₜ until a third time t_{end} to feed an auxiliary device (16) connected to the battery (12), said second time tₛₜₐᵣₜ corresponding to the moment when the battery (12) becomes in contact with the liquid; and
using a delivery energy time interval tₒₚₑᵣₐₜᵢₒₙ of the battery (12) comprised between a time tₒₙ in which a voltage output of the battery (12) is above a threshold voltage and a time t_{off} in which the voltage output is below the threshold voltage to control the duration of an event including a selective activation and deactivation of said auxiliary device (16) connected to the battery (12),
wherein the battery (12) being sized and composed to provide a given amount of energy providing the cited delivery energy time interval tₒₚₑᵣₐₜᵢₒₙ.

2. The method of claim 1, wherein the battery (12) comprises a paper-based battery having a paper part placed in contact with an oxidizing anode comprising redox species, metal, alloys or polymers, and a reducing cathode comprising an air-breathing cathode, redox species, metal, alloys or polymers.

3. The method of previous claims, wherein the microfluidic device comprises a microfluidic analytical device including a lateral flow assay device further including a sample pad (11) located at the first end and a lateral flow test strip (10) through which the liquid flows by capillarity, the liquid comprising a liquid sample.

4. The method of any of previous claims, wherein the auxiliary device (16) when activated during the delivery energy time interval tₒₚₑᵣₐₜᵢₒₙ indicating an enabling time in which a result of an assay has to be taken.

5. The method of claim 1 or 2, further comprising adjusting the delivery energy time interval tₒₚₑᵣₐₜᵢₒₙ by using one or more of the following strategies: connecting an electric discharge load (15) to the battery (12), modifying an active area of the battery (12) and/or modifying a thickness of the anode of the battery (12).

6. The method of claim 1 or 5, further comprising using an electrical circuit to switch on/off the delivering of power of the battery (12) when a given voltage level is reached.

7. The method of claim 1 wherein comprising an adjustable time interval t_{delay} between said first time t₀ and the delivery energy time interval tₒₚₑᵣₐₜᵢₒₙ.

8. A timer microfluidic device, comprising:
a first end adapted to receive an amount of liquid at a first time t₀, the microfluidic device having a second end towards the liquid flows by capillarity, and
a battery (12) configured to produce energy when in contact with the liquid, from a second time tₛₜₐᵣₜ until a third time t_{end} to feed an auxiliary device (16) connected to the battery (12),
wherein the battery (12) being sized and composed to provide a given amount of energy to control the duration of an event including a selective activation and deactivation of said auxiliary device (16) connected to the battery (12), during a delivery energy time interval tₒₚₑᵣₐₜᵢₒₙ of the battery (12) comprised between a time tₒₙ in which a voltage output of the battery (12) is above a threshold voltage and a time t_{off} in which the voltage output is below the threshold voltage.

9. The device of claim 8, wherein the battery (12) comprises a paper-based battery having a paper part placed in contact with an oxidizing anode comprising redox species, metal, alloys or polymers, and a reducing cathode comprising an air-breathing cathode, redox species, metal, alloys or polymers.

10. The device of claim 8 or 9, wherein the microfluidic device comprises a lateral flow assay device comprising a sample pad (11) located at the first end and a lateral flow test strip (10) through which the liquid (1) flows by capillarity, the liquid comprising a liquid sample.

11. The device of any of the claims 8 to 10, wherein the auxiliary device (16) comprises a lighting system including a Light Emitting Diode, LED, an audible system including a loudspeaker, a buzzer or an alarm, and/or a device transmitting a radiofrequency signal.

12. The device of any of the claims 8 to 10, wherein the auxiliary device (16) comprises a window being enabled for a vision there through during said delivery energy time interval tₒₚₑᵣₐₜᵢₒₙ and being disabled thereafter, wherein said window comprises a mechanical window, a liquid crystal dispersion film or an electrochromic film.

13. The device of any of the claims 8 to 10, wherein the auxiliary device (16) comprises a heater, said heater being heated up until a given temperature during said delivery energy time interval tₒₚₑᵣₐₜᵢₒₙ.

14. The device of claim 10, wherein the microfluidic device being placed inside a container (1).

15. The device of claim 14, wherein the container further integrates several additional devices to cooperate with the lateral flow test strip (10) including an electrical discharge load including a resistor or a digital or analog circuit, as well as switches.
